(19) Europäisches Patentamt
European Patent Office
Office européen des brevets

(11) EP 4 707 318 A1

(12) **EUROPEAN PATENT APPLICATION**
published in accordance with Art. 153(4) EPC

(43) Date of publication:
**11.03.2026 Bulletin 2026/11**

(21) Application number: **24807126.8**

(22) Date of filing: **10.05.2024**

(51) International Patent Classification (IPC):
***C08F 290/06*** (2006.01) ***B24B 37/00*** (2012.01)
***C08F 112/14*** (2006.01) ***C08F 212/14*** (2006.01)
***C08F 222/38*** (2006.01) ***C08F 297/00*** (2006.01)
***C08F 299/02*** (2006.01) ***C09K 3/14*** (2006.01)
***H01L 21/304*** (2006.01)

(52) Cooperative Patent Classification (CPC):
**B24B 37/00; C08F 112/14; C08F 212/14; C08F 222/38; C08F 290/06; C08F 297/00; C08F 299/02; C09K 3/14; H10P 52/00**

(86) International application number:
**PCT/JP2024/017333**

(87) International publication number:
**WO 2024/237174 (21.11.2024 Gazette 2024/47)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **17.05.2023 JP 2023081291**
**17.05.2023 JP 2023081292**

(71) Applicant: **Toagosei Co., Ltd.**
**Minato-ku**
**Tokyo 105-8419 (JP)**

(72) Inventors:
- **FUJIMOTO Yuya**
**Tokyo 105-8419 (JP)**
- **KANBE Shinya**
**Tokyo 105-8419 (JP)**
- **SANAI Yasuyuki**
**Tokyo 105-8419 (JP)**
- **ISO Takamasa**
**Tokyo 105-8419 (JP)**

(74) Representative: **Kramer Barske Schmidtchen Patentanwälte PartG mbB**
**European Patent Attorneys**
**Landsberger Strasse 300**
**80687 München (DE)**

(54) **VINYL-BASED POLYMER, METHOD FOR PRODUCING SAME, POLISHING LIQUID, AND VINYL-BASED MONOMER**

(57) A vinyl-based polymer contains a structural unit (A) derived from a vinyl-based monomer (A) represented by the following structural formula (1).

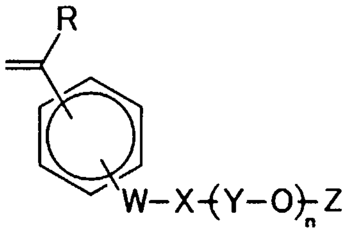

・・・(1)

In the structural formula (1), R represents a hydrogen atom or a methyl group, W represents a divalent hydrocarbon group having 6 or less carbon atoms, X represents a heteroatom-containing divalent functional group having 1 or less carbon atoms, Y represents a divalent hydrocarbon group having 6 or less carbon atoms, Z represents a hydrogen atom or an alkyl group having 1 or more and 4 or less carbon atoms, and n represents an integer of 4 or more. The vinyl-based polymer is obtained, for example, by polymerizing one or more types of vinyl-based monomers including the vinyl-based monomer (A) by a living radical polymerization method.

EP 4 707 318 A1

## Description

### TECHNICAL FIELD

The present invention relates to a vinyl-based polymer, a method for producing the same, a polishing liquid, and a vinyl-based monomer.

### BACKGROUND ART

Semiconductor devices are used for almost all electronic devices in daily life, such as information communication devices and home electric appliances, and are thus indispensable for modern life. In recent years, the role played by the semiconductor devices has further increased due to the spread of IoT, the utilization of cloud, and the like. Hitherto, high integration and large capacity of semiconductor chips have been achieved at a significant speed, but the demand for high performance has no end, and the importance of fine processing technology is still increasing.

In order to realize high performance of semiconductor devices, a step of polishing a surface of a silicon wafer to a desired quality, such as a polishing step of polishing a surface of a silicon substrate to become a high quality surface, or a chemical mechanical polishing (CMP) step of polishing an insulating layer and/or a wiring layer formed on a silicon substrate, is extremely important. Therefore, for the purpose of improving processing accuracy in these polishing steps, polishing liquids containing various watersoluble polymers are used.

For example, in the CMP step, a polishing liquid composition containing a (meth)acrylic acid ester-based polymer (see Patent Literature 1) and the like are used.

### PRIOR ART LITERATURE

#### Patent Literature

Patent Literature 1: JP-A-2017-17177

### SUMMARY OF INVENTION

### PROBLEMS TO BE SOLVED BY INVENTION

However, the (meth)acrylic acid ester-based polymer contains an ester bond in the molecular structure. The ester bond has hydrolyzability. Accordingly, for example, when the ester bond is hydrolyzed while the polishing liquid is stored, the characteristics of the polishing liquid are changed. This results in a possibility that desired processing accuracy cannot be obtained.

The present invention has been made in view of such a background, and an object of the present invention is to provide a vinyl-based polymer that can be used as an additive for a polishing liquid and has excellent storage stability, a method for producing the vinyl-based polymer, a polishing liquid containing the vinyl-based polymer, and a vinyl-based monomer used as a raw material for the vinyl-based polymer.

### MEANS FOR SOLVING PROBLEM

A first aspect of the present invention is a vinyl-based polymer according to any one of the following [1] to [10].

[1] A vinyl-based polymer containing a structural unit (A) derived from a vinyl-based monomer (A) represented by the following structural formula (1).

[Chemical Formula 1]

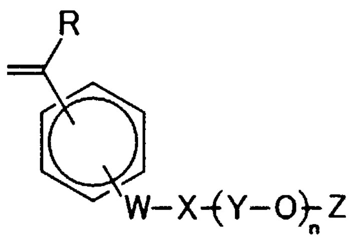

・・・(1)

In the structural formula (1), R represents a hydrogen atom or a methyl group, W represents a divalent hydrocarbon group having 6 or less carbon atoms, X represents a heteroatom-containing divalent functional group having 1 or less carbon atoms, Y represents a divalent hydrocarbon group having 6 or less carbon atoms, Z represents a hydrogen atom or an alkyl group having 1 or more and 4 or less carbon atoms, and n represents an integer of 4 or more.
[2] The vinyl-based polymer according to [1], wherein the vinyl-based polymer is composed of the structural unit (A), and the structural unit (A) is derived from a vinyl-based monomer (A') represented by the following structural formula (1').

[Chemical Formula 2]

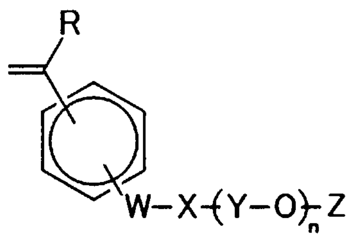

$\cdots$(1')

In the structural formula (1'), R represents a hydrogen atom or a methyl group, W represents a divalent hydrocarbon group having 6 or less carbon atoms, X represents a heteroatom-containing divalent functional group having 1 or less carbon atoms, Y represents a divalent hydrocarbon group having 6 or less carbon atoms, Z represents a hydrogen atom or an alkyl group having 1 or more and 4 or less carbon atoms, and n represents an integer of 6 or more.
[3] The vinyl-based polymer according to [1], wherein the vinyl-based polymer contains the structural unit (A) and a structural unit (B) derived from a vinyl-based monomer (B) different from the vinyl-based monomer (A).
[4] The vinyl-based polymer according to [3], wherein the vinyl-based polymer contains the structural unit (B) having an SP value of 17 $(J/cm^3)^{0.5}$ or more and 25 $(J/cm^3)^{0.5}$ or less as calculated by Fedors' estimation method.
[5] The vinyl-based polymer according to [3] or [4], wherein the vinyl-based monomer (B) contains a (meth) acrylamide-based monomer.
[6] The vinyl-based polymer according to any one of [3] to [5], wherein the vinyl-based polymer is a block copolymer having a polymer block (A) containing the structural unit (A) and a polymer block (B) containing the structural unit (B).
[7] The vinyl-based polymer according to [6], wherein the polymer block (B) is composed of the structural unit (B) having an SP value of 17 $(J/cm^3)^{0.5}$ or more and 25 $(J/cm^3)^{0.5}$ or less as calculated by Fedors' estimation method.
[8] The vinyl-based polymer according to [6] or [7], wherein the vinyl-based polymer is a diblock copolymer having the polymer block (A) and the polymer block (B), and a ratio of the polymer block (A) to a total of the polymer block (A) and the polymer block (B) is 0.1 mass% or more and 99.9 mass% or less.
[9] The vinyl-based polymer according to any one of [1] to [8], wherein the vinyl-based polymer has a number average molecular weight Mn of 1,000 or more and 1,000,000 or less.
[10] The vinyl-based polymer according to any one of [1] to [9], wherein a dispersity index PDI, as expressed by the following equation (I), of the vinyl-based polymer is 2.0 or less:

PDI = weight average molecular weight Mw/number average molecular weight Mn (I).

A second aspect of the present invention is a method for producing a vinyl-based polymer according to the following [11] or [12].
[11] A method for producing the vinyl-based polymer according to any one of [1] to [10], the method including polymerizing one or more monomers including the vinyl-based monomer (A) by a living radical polymerization method to produce the vinyl-based polymer.
[12] The method for producing a vinyl-based polymer according to [11], wherein the living radical polymerization method is a reversible addition-fragmentation chain transfer polymerization method.
A third aspect of the present invention is a polishing liquid according to any one of the following [13] to [16].
[13] A polishing liquid containing the vinyl-based polymer according to any one of [1] to [10].
[14] The polishing liquid according to [13], wherein the polishing liquid is configured to be used for chemical mechanical polishing.
[15] The polishing liquid according to [13] or [14], wherein the polishing liquid is configured to be used for planarization of a surface of an insulating layer and/or a wiring layer in a semiconductor device.
[16] The polishing liquid according to any one of [13] to [15], wherein the polishing liquid contains one or two types of abrasive grains selected from the group consisting of ceria and silica.

A fourth aspect of the present invention is a vinyl-based monomer according to the following [17].
[17] A vinyl-based monomer represented by the following structural formula (1').

[Chemical Formula 3]

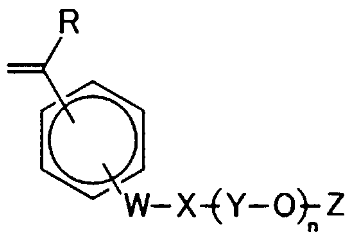

...(1')

In the structural formula (1'), R represents a hydrogen atom or a methyl group, W represents a hydrocarbon group having 6 or less carbon atoms, X represents a heteroatom-containing divalent functional group having 1 or less carbon atoms, Y represents a divalent hydrocarbon group having 6 or less carbon atoms, Z represents an alkyl group having 1 or more and 4 or less carbon atoms, and n represents an integer of 6 or more.

EFFECTS OF INVENTION

The above aspects can provide a vinyl-based polymer that can be used as an additive for a polishing liquid and has excellent storage stability, a method for producing the vinyl-based polymer, a polishing liquid containing the vinyl-based polymer, and a vinyl-based monomer used as a raw material for the vinyl-based polymer.

MODE FOR CARRYING OUT INVENTION

(Vinyl-based polymer)

The above vinyl-based polymer (hereinafter, referred to as the "polymer") contains a structural unit (A) derived from a vinyl-based monomer (A). The vinyl-based polymer may consist only of the structural unit (A), for example. Further, the vinyl-based polymer may contain the structural unit (A) and a structural unit (B) derived from a vinyl-based monomer (B) different from the vinyl-based monomer (A). Hereinafter, each structural unit contained in the polymer will be described.

[Structural unit (A)]

The structural unit (A) contained in the polymer is a structural unit derived from the vinyl-based monomer (A) represented by the following structural formula (1). All of the structural units (A) contained in the polymer may have the same structure. Further, two or more types of structural units (A) having different structures may be contained in the polymer. More specifically, the polymer may have only a structural unit (A) where R, W, X, Y, Z, and n in the following structural formula (1) are the same, or may have a plurality of types of structural units (A) where at least one of R, W, X, Y, Z, and n is different.

[Chemical Formula 4]

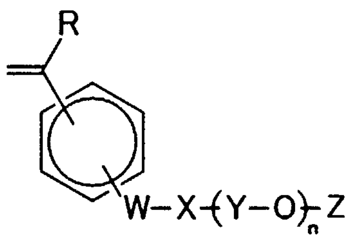

...(1)

R in the structural formula (1) is a hydrogen atom or a methyl group. W in the structural formula (1) is a divalent hydrocarbon group having 6 or less carbon atoms. Examples of W in the structural formula (1) include alkylene groups such as a methylene group, an ethylene group, a $-CH(CH_3)-$ group, a $-C(CH_3)_2-$ group, and an n-propylene group.
X in the structural formula (1) is a heteroatom-containing divalent functional group having 1 or less carbon atoms.

Examples of X in the structural formula (1) include an ether group (-O-), a urea bond (-NHC(=O)NH-), and a urethane bond (-NHC(=O)O-).

X in the structural formula (1) is preferably a functional group not containing an ester bond, and more preferably an ether group. In this case, the storage stability of the polymer can be further improved. When X in the structural formula (1) is an ether group, W in the structural formula (1) is preferably a methylene group.

Y in the structural formula (1) is a divalent hydrocarbon group having 6 or less carbon atoms. Examples of Y in the structural formula (1) include saturated hydrocarbon groups such as a methylene group, an ethylene group, a -CHMe- group, an n-propylene group, a -CHEt- group, a $-CHMeCH_2-$ group, a $-CH_2CHMe-$ group, an n-butylene group, a -CH(n-Pr)- group, a -CH(i-Pr)- group, a $-CHEtCH_2-$ group, a $-CH_2CHEt-$ group, a - $CHMeCH_2CH_2-$ group, a $-CH_2CHMeCH_2-$ group, and a $-CH_2CH_2CHMe-$ group. A plurality of Y in the structural formula (1) may have the same structure. In addition, the structural formula (1) may include two or more types of Y having different structures.

In consideration of industrial availability of raw materials and solubility of the polymer in water, Y in the structural formula (1) is preferably a saturated hydrocarbon group, more preferably one or more saturated hydrocarbon groups selected from the group consisting of an ethylene group, a $-CHMeCH_2-$ group, and a $-CH_2CHMe-$ group, and still more preferably an ethylene group.

n in the structural formula (1) is an integer of 4 or more. n in the structural formula (1) is preferably an integer of 6 or more. In this case, the water solubility of the polymer can be easily adjusted to a range suitable for a polishing liquid. On the other hand, from the viewpoint of storage stability, the upper limit of n in the structural formula (1) is not particularly limited. However, when the value of n is excessively large, in a case where the polymer is used as an additive for a polishing liquid, a decrease in the removal rate may be caused. From the viewpoint of more easily avoiding such a problem, the value of n in the structural formula (1) is preferably 150 or less, more preferably 100 or less, still more preferably 50 or less, and particularly preferably 30 or less.

In configuring the preferred range of the value of n in the structural formula (1), the upper limit and the lower limit of the value of n described above can be arbitrarily combined. For example, the preferred range of the value of n in the structural formula (1) may be 4 or more and 150 or less, 4 or more and 100 or less, 4 or more and 50 or less, or 4 or more and 30 or less. In addition, the preferred range of the value of n in the structural formula (1) may be 6 or more and 150 or less, 6 or more and 100 or less, 6 or more and 50 or less, or 6 or more and 30 or less. In particular, when the polymer consists only of the structural unit (A), n in the structural formula (1) is preferably 6 or more, more preferably 6 or more and 150 or less, still more preferably 6 or more and 100 or less, particularly preferably 6 or more and 50 or less, and most preferably 6 or more and 30 or less.

It is noted that n in the structural formula (1) is the total number of repeating units -(Y-O)- present between X and Z in the structural formula (1). Thus, for example, when all of Y in the structural formula (1) have the same structure, n in the structural formula (1) is equal to the number of Y. Also, for example, when two or more types of Y having different structures are included in the structural formula (1), n in the structural formula (1) is equal to the sum of the numbers of Y.

Z in the structural formula (1) is a hydrogen atom or an alkyl group having 1 or more and 4 or less carbon atoms. For example, when Z in the structural formula (1) is an alkyl group, Z may be a methyl group, an ethyl group, an n-propyl group, an i-propyl group, an n-butyl group, an i-butyl group, a *sec*-butyl group, a tert-butyl group, or the like. In consideration of industrial availability of raw materials and solubility of the polymer in water, Z in the polymer is preferably a methyl group.

When the polymer contains a structural unit (A) and a structural unit (B), the content of the structural unit (A) in the polymer is preferably 0.1 mass% or more and 99.9 mass% or less, more preferably 30 mass% or more and 99 mass% or less, still more preferably 50 mass% or more and 98 mass% or less, particularly preferably 80 mass% or more and 97 mass% or less, and most preferably 91 mass% or more and 97 mass% or less, with respect to the total content of the structural unit (A) and the structural unit (B).

When the polymer, which has a ratio between the structural unit (A) and the structural unit (B) within the above-mentioned specific range, is used as an additive for a polishing liquid, the polymer tends to be adsorbed onto an oxide film and exhibit a protective action. In addition, such a polymer is highly responsive to a change in polishing pressure. When the oxide film is a convex portion (in a state where the polishing pressure is high), the polymer is not adsorbed and a decrease in the removal rate can be suppressed. At the same time, when the polishing progresses, a nitride film is exposed, and the polishing target becomes a concave portion (in a state where the polishing pressure is low), the polymer is adsorbed onto the oxide film interface and excessive polishing tends to be suppressed. Accordingly, it is considered that by setting the ratio between the structural unit (A) and the structural unit (B) within the specific range, a favorable polished surface with suppressed dishing can be easily obtained without lowering the removal rate. Therefore, a polymer having a ratio between the structural unit (A) and the structural unit (B) of within the specific range is suitable as an additive for the polishing liquid.

[Structural unit (B)]

The polymer may contain a structural unit (B). The structural unit (B) contained in the polymer is a structural unit derived from a vinyl-based monomer (B) having a structure different from the vinyl-based monomer (A). The polymer may

contain only the structural unit (B) derived from one type of vinyl-based monomer (B), or may contain the structural unit (B) derived from two or more types of vinyl-based monomers (B).

As a vinyl-based monomer (B), a monomer other than the vinyl-based monomer (A) among monomers having a vinyl group can be used. The vinyl-based monomer (B) preferably has a structure that is relatively difficult to hydrolyze. Examples of the monomer having such a structure include a (meth)acrylamide-based monomer, a vinyl ether-based monomer, and a styrene-based monomer. By using these monomers as the vinyl-based monomer (B), deterioration of the storage stability of the polymer can be avoided. Among them, the polymer containing the structural unit (B) derived from the (meth)acrylamide-based monomer can easily adjust the balance between hydrophilicity and hydrophobicity to an appropriate range when it is used as an additive for a polishing liquid. Therefore, the polymer can be suitably adsorbed onto the oxide film interface, and excessive polishing can be suppressed.

Examples of the (meth)acrylamide-based monomer that can be used include (meth)acrylamide, (meth)acryloyl morpholine, and alkyl (meth)acrylamide. Examples of the alkyl (meth)acrylamide include tert-butyl (meth)acrylamide, N,N-dimethyl (meth)acrylamide, N,N-diethyl (meth)acrylamide, N-isopropyl (meth)acrylamide, N,N-dimethylaminopropyl (meth)acrylamide, and N-(2-hydroxyethyl)acrylamide. The (meth)acrylamide-based monomer is preferably tert-butyl acrylamide and/or N-isopropyl (meth)acrylamide, and more preferably N-isopropyl (meth)acrylamide.

Examples of the vinyl ether-based monomer that can be used include alkyl vinyl ethers such as methyl vinyl ether, ethyl vinyl ether, n-propyl vinyl ether, isopropyl vinyl ether, n-butyl vinyl ether, isobutyl vinyl ether, t-butyl vinyl ether, n-hexyl vinyl ether, 2-ethylhexyl vinyl ether, n-octyl vinyl ether, n-nonyl vinyl ether, and n-decyl vinyl ether.

Examples of the styrene-based monomer that can be used include aromatic vinyl compounds such as styrene, vinyl toluene, and vinyl xylene.

It is noted that the "(meth)acrylamide-based monomer" described above is a concept including an amide of acrylic acid and a derivative thereof and an amide of methacrylic acid and a derivative thereof. That is, for example, the "(meth)acrylamide" is a concept including acrylamide and methacrylamide. The "alkyl (meth)acrylamide" is a concept including alkyl acrylamide and alkyl methacrylamide, and the "(meth)acryloyl morpholine" is a concept including acryloyl morpholine and methacryloyl morpholine.

The content of the structural unit derived from the (meth)acrylamide-based monomer in the polymer is preferably 0.1 mass% or more, more preferably 1 mass% or more, still more preferably 2 mass% or more, and particularly preferably 3 mass% or more, with respect to the total content of the structural unit (A) and the structural unit (B). The content of the structural unit derived from the (meth)acrylamide-based monomer in the polymer is preferably 99.9 mass% or less, more preferably 70 mass% or less, still more preferably 50 mass% or less, particularly preferably 20 mass% or less, and most preferably 7 mass% or less, with respect to the total content of the structural unit (A) and the structural unit (B). In this case, when the polymer is used as an additive for a polishing liquid, a favorable polished surface with suppressed dishing can be easily obtained.

In configuring the preferred range of the content of the structural unit derived from the (meth)acrylamide-based monomer in the polymer, the upper limit and the lower limit of the content of the structural unit derived from the (meth) acrylamide-based monomer described above can be arbitrarily combined. For example, the preferred range of the content of the structural unit derived from the (meth)acrylamide-based monomer in the polymer may be 0.1 mass% or more and 99.9 mass% or less, 1 mass% or more and 70 mass% or less, 2 mass% or more and 50 mass% or less, 3 mass% or more and 20 mass% or less, or 3 mass% or more and 7 mass% or less.

In addition, examples of the vinyl-based monomer (B) that can also be used include (meth)acrylic acid esters such as methyl (meth)acrylate, ethyl (meth)acrylate, n-propyl (meth)acrylate, i-propyl (meth)acrylate, n-butyl (meth) acrylate, and t-butyl (meth)acrylate); vinyl alcohols such as vinyl alcohol, 2-hydroxyethyl vinyl ether, diethylene glycol monovinyl ether, and 4-hydroxybutyl vinyl ether; and $\alpha$-olefins such as ethylene, propylene, and butylene.

The content of the structural unit derived from the vinyl-based monomer (B) other than the (meth)acrylamide-based monomer in the polymer is preferably 10 mass% or less, more preferably 8 mass% or less, still more preferably 5 mass% or less, still even more preferably 3 mass% or less, particularly preferably 1 mass% or less, and most preferably 0 mass%, that is, all the structural units (B) in the polymer are structural units derived from the (meth)acrylamide-based monomer.

The polymer preferably contains a structural unit (B) having an SP value, as calculated by Fedors' estimation method (see, for example, Polymer Engineering & Science, Vol. 14, No. 2, pp. 147-154, published in 1974), of 17 $(J/cm^3)^{0.5}$ or more and 25 $(J/cm^3)^{0.5}$ or less, and more preferably contains a structural unit (B) having an SP value of 18 $(J/cm^3)^{0.5}$ or more and 21.8 $(J/cm^3)^{0.5}$ or less. The polymer containing such a structural unit (B) can enhance the processing accuracy when it is used as an additive for a polishing liquid. From the same viewpoint, the SP values of all the structural units (B) in the polymer, as calculated by Fedors' estimation method, are more preferably 17 $(J/cm^3)^{0.5}$ or more and 25 $(J/cm^3)^{0.5}$ or less, and particularly preferably 18 $(J/cm^3)^{0.5}$ or more and 21.8 $(J/cm^3)^{0.5}$ or less.

Examples of the structural unit (B) having an SP value in the specific range that can be preferably used include a structural unit derived from tert-butyl acrylamide and a structural unit derived from N-isopropyl acrylamide.

[Molecular weight of polymer]

The number average molecular weight Mn of the polymer can be appropriately set within a range of 1,000 or more and 1,000,000 or less. When the polymer is used as an additive for a polishing liquid, the number average molecular weight of the polymer is preferably 1,500 or more, more preferably 2,000 or more, still more preferably 3,000 or more, and particularly preferably 4,000 or more. In this case, it is possible to suppress a decrease in the removal rate while sufficiently securing the wettability of the surface of a polishing target.

When the polymer is used as an additive for a polishing liquid, the number average molecular weight of the polymer is preferably 100,000 or less, more preferably 60,000 or less, still more preferably 30,000 or less, and particularly preferably 10,000 or less. In this case, aggregation of abrasive grains due to a shearing force can be sufficiently suppressed, and occurrence of defects such as scratches during polishing can be sufficiently suppressed.

In configuring the preferred range of the number average molecular weight Mn of the polymer, the upper limit and the lower limit of the number average molecular weight Mn of the polymer described above can be arbitrarily combined. For example, the preferred range of the number average molecular weight Mn of the polymer may be 1,000 or more and 60,000 or less, 1,500 or more and 30,000 or less, 2,000 or more and 30,000 or less, 2,500 or more and 10,000 or less, or 4,000 or more and 10,000 or less.

The dispersity index PDI of the vinyl-based polymer as expressed by the following equation (I) is preferably 2.0 or less, more preferably 1.8 or less, still more preferably 1.5 or less, particularly preferably 1.3 or less, and most preferably 1.2 or less. It is noted that the lower limit of the dispersity index PDI is usually 1.0.

PDI = weight average molecular weight Mw/number average molecular weight Mn (I).

The polymer having a dispersity index PDI within the above specific range has characteristics suitable as an additive for a polishing liquid. As a reason for this, for example, the following reasons are conceivable. In a polymer having a function of dispersing abrasive grains, it is considered that the magnitude of the molecular weight affects the rate of adsorption and desorption on a polishing target, and it is generally considered that the smaller the molecular weight of the polymer, the higher the rate of adsorption-desorption on the polishing target. In addition, it is considered that a polymer having a large molecular weight is likely to form an aggregate structure of abrasive grains due to a shearing force. Therefore, in the polymer having a function of dispersing abrasive grains, it is preferable that the molecular weight distribution is narrow.

It is noted that the number average molecular weight Mn and the weight average molecular weight Mw of the polymer herein are values in terms of polymethyl methacrylate as obtained by converting the retention time measured by gel permeation chromatography (GPC) into the molecular weight while using standard polymethyl methacrylate. More specific measurement conditions for measuring the molecular weight will be described in Examples.

[Block copolymer]

When the polymer contains a structural unit (A) and a structural unit (B), the polymer may be a random copolymer containing the structural unit (A) and the structural unit (B), but is preferably a block copolymer having a polymer block (A) containing the structural unit (A) and a polymer block (B) containing the structural unit (B). In the random copolymer, functional groups to be adsorbed onto the surface of a polishing target such as a silicon wafer are uniformly disposed on the entire molecular chain. On the other hand, the block copolymer has a region in which functional groups to be adsorbed onto the surface of the polishing target are arranged in a concentrated manner on the molecular chain. Therefore, when the block copolymer is used as an additive for a polishing liquid, the block copolymer is easily adsorbed onto the surface of the polishing target, so that the surface of the polishing target is more easily protected. As a result, excessive polishing of the polishing target can be more easily avoided.

When the polymer is a block copolymer, it is sufficient that the polymer has at least one polymer block (A) and at least one polymer block (B). More specifically, the polymer may be, for example, an AB diblock copolymer having one or more repeating units composed of a polymer block (A) and a polymer block (B). In addition, the polymer may be, for example, a triblock copolymer such as an ABA triblock copolymer having one or more repeating units composed of a polymer block (A), a polymer block (B), and a polymer block (A), or a BAB triblock copolymer having one or more repeating units composed of a polymer block (B), a polymer block (A), and a polymer block (B). In addition, the block copolymer may be a multiblock copolymer having four or more polymer blocks.

Further, the polymer may contain a polymer block (C) different from the polymer block (A) and the polymer block (B). In this case, the polymer may take a structure such as an ABC triblock copolymer having one or more repeating units composed of a polymer block (A), a polymer block (B), and a polymer block (C), or an ABCA multiblock copolymer having one or more repeating units composed of a polymer block (A), a polymer block (B), a polymer block (C), and a polymer

block (A).

Among them, the polymer is preferably a diblock copolymer (i.e., an AB diblock copolymer) having a repeating unit(s) composed of the polymer block (A) and the polymer block (B). Since the diblock copolymer is obtained by a simple production process, the possibility of contamination with various impurities is small. A high-purity polymer can thus be more easily obtained.

The ratio of the polymer block (A) to the total of the polymer block (A) and the polymer block (B) is preferably 0.1 mass% or more and 99.9 mass% or less, more preferably 30 mass% or more and 99 mass% or less, still more preferably 50 mass% or more and 98 mass% or less, particularly preferably 80 mass% or more and 97 mass% or less, and most preferably 93 mass% or more and 97 mass% or less. Such a polymer tends to be adsorbed onto an oxide film and exhibit a protective effect when it is used as an additive for a polishing liquid. In addition, such a polymer is highly responsive to a change in polishing pressure, and is not adsorbed onto an oxide film, thereby not decreasing the removal rate in a case where the oxide film is a convex portion (in a state where the polishing pressure is high); on the other hand, the polymer is adsorbed onto an oxide film interface and tends to suppress excessive polishing in a case where polishing progresses, a nitride film is exposed, and the polishing target becomes a concave portion (in a state where the polishing pressure is low). In view of the above, it is considered that by using the polymer as an additive for a polishing liquid, a favorable polished surface with suppressed dishing is easily obtained without lowering the removal rate.

In a case where the polymer contains the polymer block (A), the polymer block (B), and the polymer block (C), the total content of the polymer block (A) and the polymer block (B) in the polymer is preferably 90 mass% or more, more preferably 95 mass% or more, still more preferably 98 mass% or more, and particularly preferably 99 mass% or more.

· Polymer block (A)

The polymer block (A) is mainly composed of the structural unit (A) derived from the vinyl-based monomer (A). The polymer block (A) may contain one type of structural unit (A) or two or more types of structural units (A). In addition, the polymer block (A) may contain a structural unit other than the structural unit (A) as long as the above-described effect is not impaired. Examples of the structural unit, other than the structural unit (A), that can be contained in the polymer block (A) include structural units derived from (meth)acrylamide-based monomers, structural units derived from (meth)acrylic acid esters, structural units derived from alkyl vinyl ethers, structural units derived from vinyl alcohols, structural units derived from aromatic vinyl compounds, and structural units derived from $\alpha$-olefins.

The content of the structural unit (A) in the polymer block (A) is preferably 80 mass% or more, more preferably 90 mass% or more, still more preferably 95 mass% or more, still even more preferably 97 mass% or more, particularly preferably 99 mass% or more, and most preferably 100 mass%, that is, the polymer block (A) consists only of the structural unit (A). When such a polymer is used as an additive for a polishing liquid, the polymer is highly responsive to a change in polishing pressure, and is not adsorbed onto an oxide film, thereby suppressing a decrease in the removal rate in a case where the oxide film is a convex portion (in a state where the polishing pressure is high). In addition, the polymer is adsorbed onto an oxide film interface and can suppress excessive polishing in a case where polishing progresses, the nitride film is exposed, and the polishing target becomes a concave portion (in a state where the polishing pressure is low). In view of the above, it is considered that by using such a polymer as an additive for a polishing liquid, a favorable polished surface with suppressed dishing is easily obtained without lowering the removal rate.

The weight average molecular weight of the polymer block (A) is preferably 500 or more, more preferably 900 or more, still more preferably 1,500 or more, particularly preferably 2,100 or more, and most preferably 2,700 or more. In addition, the weight average molecular weight of the polymer block (A) is preferably 100,000 or less, more preferably 60,000 or less, still more preferably 30,000 or less, particularly preferably 10,000 or less, and most preferably 6,000 or less. By using the polymer having a weight average molecular weight of the polymer block (A) within the above-described range as an additive for a polishing liquid, it is possible to suppress a decrease in the removal rate while sufficiently securing the wettability of the surface of a polishing target. In addition, aggregation of abrasive grains due to a shearing force can be sufficiently suppressed, and occurrence of defects such as scratches during polishing can be sufficiently suppressed.

In configuring the preferred range of the weight average molecular weight of the polymer block (A), the lower limit and the upper limit of the weight average molecular weight of the polymer block (A) described above can be arbitrarily combined. For example, the preferred range of the weight average molecular weight of the polymer block (A) may be 500 or more and 100,000 or less, 900 or more and 60,000 or less, 1,500 or more and 30,000 or less, 2,100 or more and 10,000 or less, or 2,700 or more and 6,000 or less.

· Polymer block (B)

The polymer block (B) is mainly composed of a structural unit (B) derived from the vinyl-based monomer (B). The polymer block (B) may contain one type of structural unit (B) or two or more types of structural units (B). In addition, the polymer block (B) may contain a structural unit other than the structural unit (B) as long as the above-described effect is not

impaired. Examples of the structural unit, other than the structural unit (B), that can be contained in the polymer block (B) include structural units derived from the vinyl-based monomers represented by the structural formula (1).

The content of the structural unit (B) in the polymer block (B) is preferably 80 mass% or more, more preferably 90 mass% or more, still more preferably 95 mass% or more, still even more preferably 97 mass% or more, and particularly preferably 99 mass% or more.

In addition, from the viewpoint of more reliably obtaining a polymer having characteristics suitable as an additive for a polishing liquid, the content of the structural unit derived from the (meth)acrylamide-based monomer in the polymer block (B) is preferably 90 mass% or more, more preferably 92 mass% or more, still more preferably 95 mass% or more, still even more preferably 97 mass% or more, and particularly preferably 99 mass% or more.

The weight average molecular weight of the polymer block (B) is preferably 100 or more, more preferably 120 or more, still more preferably 130 or more, particularly preferably 140 or more, and most preferably 150 or more. In addition, the weight average molecular weight of the polymer block (B) is preferably 50,000 or less, more preferably 10,000 or less, still more preferably 5,000 or less, particularly preferably 1,000 or less, and most preferably 500 or less.

In configuring the preferred range of the weight average molecular weight of the polymer block (B), the lower limit and the upper limit of the weight average molecular weight of the polymer block (B) described above can be arbitrarily combined. For example, the preferred range of the weight average molecular weight of the polymer block (B) may be 100 or more and 50,000 or less, 120 or more and 10,000 or less, 130 or more and 5,000 or less, 140 or more and 1,000 or less, or 150 or more and 500 or less.

In addition, the polymer block (B) is preferably composed of the structural unit (B) having an SP value of 17 $(J/cm^3)^{0.5}$ or more and 25 $(J/cm^3)^{0.5}$ or less as calculated by Fedors' estimation method, and more preferably composed of the structural unit (B) having an SP value of 18 $(J/cm^3)^{0.5}$ or more and 21.8 $(J/cm^3)^{0.5}$ or less. The polymer containing such a structural unit can further enhance the processing accuracy when it is used as an additive for a polishing liquid.

(Method for producing vinyl-based polymer)

The method for producing the polymer is not particularly limited as long as the effect of the present invention is not impaired. For example, the polymer can be produced by polymerizing one or more types of monomers including the vinyl-based monomer (A) represented by the structural formula (1) by employing a known radical polymerization method such as a solution polymerization method or bulk polymerization. In a case of synthesizing a vinyl-based polymer by a solution polymerization method, for example, a solvent and a monomer are charged into a reactor, a polymerization initiator is added, and the mixture is heated to polymerize the monomer, whereby a target polymer can be obtained.

In addition, in order to adjust the molecular weight distribution and the like of the polymer, the polymer obtained by the above-described method may be purified, as necessary. As a method for purifying the polymer, for example, a known polymer purification method such as a reprecipitation method or a method using a porous material can be adopted.

Examples of suitable methods for producing the polymer include various controlled polymerization methods such as living radical polymerization and living anion polymerization. Among them, it is preferable to obtain the vinyl-based polymer by polymerizing one or more types of monomers including the vinyl-based monomer (A) by a living radical polymerization method from the viewpoints that a polymer having high controllability of the dispersity index (PDI) of the molecular weight and excellent dispersion stability of abrasive grains can be produced, and that the operation is simple and the method is applicable to a wide range of monomers. When the living radical polymerization method is employed, the polymerization mode is not particularly limited, and the polymerization can be performed by various modes such as bulk polymerization, solution polymerization, emulsion polymerization, mini-emulsion polymerization, and suspension polymerization.

For example, when a living radical polymerization method is employed and the polymer is produced by solution polymerization, the polymer can be obtained by charging a solvent and a monomer into a reactor, adding a radical polymerization initiator, and performing polymerization preferably by heating. In the polymerization, any process such as a batch process, a semi-batch process, a dry continuous polymerization process, or a continuous stirred-tank reactor process (CSTR) may be adopted.

In the production of the polymer, as the living radical polymerization method, a polymerization method using a known polymerization mechanism can be employed. As the living radical polymerization methods, a living radical polymerization method using an exchange chain mechanism, a living radical polymerization method using an combination-dissociation mechanism, and a living radical polymerization method using an atom transfer mechanism can be employed. More specifically, examples of the living radical polymerization using an exchange chain mechanism that can be adopted include a reversible addition-fragmentation chain transfer polymerization method (RAFT method), an iodine transfer polymerization method, a polymerization method using an organic tellurium compound (TERP method), a polymerization method using an organic antimony compound (SBRP method), or a polymerization method using an organic bismuth compound (BIRP method). Examples of the living radical polymerization using an association-dissociation mechanism that can be adopted include a nitroxy radical method (NMP method). Examples of the living radical

polymerization using an atom transfer mechanism that can be adopted include an atom transfer radical polymerization method (ATRP method). Among them, it is preferable to employ a living radical polymerization method using an exchange chain mechanism from the viewpoint of being applicable to the widest range of monomers and having excellent controllability of polymerization. In addition, it is preferable to employ the RAFT method or the NMP method from the viewpoint of being able to avoid contamination of a polishing target by metal or semimetal compounds. Further, it is particularly preferable to employ the RAFT method from the viewpoint of easy synthesis in an aqueous system without requiring a high temperature.

In the RAFT method, polymerization proceeds via a reversible chain transfer reaction in the presence of a polymerization control agent (RAFT agent) and a radical polymerization initiator. Examples of the RAFT agents that can be used include various known RAFT agents such as dithioester compounds, xanthate compounds, trithiocarbonate compounds, and dithiocarbamate compounds. Among them, trithiocarbonate compounds and dithiocarbamate compounds are preferable because a polymer having a smaller dispersity index of the molecular weight can be obtained. In addition, it is possible to use, as the RAFT agent, a monofunctional compound having only one active point or a polyfunctional compound having two or more active points. The amount of the RAFT agent used is appropriately adjusted depending on, for instance, the types of the monomer and the RAFT agent used.

As the radical polymerization initiator to be used in the polymerization by the RAFT method, known radical polymerization initiators such as azo compounds, organic peroxides, and persulfates can be used. Among these, an azo compound is preferable because it is easy to handle in terms of safety, and a side reaction during radical polymerization is less likely to occur. Specific examples of the azo compound include 2,2'-azobis(isobutyronitrile), 2,2'-azobis(2,4-dimethylvaleronitrile), 4,4'-azobis(4-cyanovaleric acid), 2,2'-azobis(4-methoxy-2,4-dimethylvaleronitrile), dimethyl-2,2'-azobis(2-methylpropionate), 2,2'-azobis(2-methylbutyronitrile), 1,1'-azobis(cyclohexane-1-carbonitrile), 2,2'-azobis[N-(2-propenyl)-2-methylpropionamide], and 2,2'-azobis(N-butyl-2-methylpropionamide). Only one type of these radical polymerization initiators may be used, or two or more types of the radical polymerization initiators may be used in combination.

The amount of the radical polymerization initiator used is preferably 0.5 mol or less and more preferably 0.2 mol or less with respect to 1 mol of the RAFT agent, from the viewpoint of further decreasing the dispersity index PDI of the molecular weight of the polymer. In addition, from the viewpoint of stably performing the polymerization reaction, the amount of the radical polymerization initiator used is preferably 0.01 mol or more, and more preferably 0.05 mol or more with respect to 1 mol of the RAFT agent. The amount of the radical polymerization initiator used per 1 mol of the RAFT agent is preferably 0.01 mol or more and 0.5 mol or less, and more preferably 0.05 mol or more and 0.2 mol or less.

When a solvent is used in living radical polymerization, examples of the polymerization solvent include aromatic compounds such as benzene, toluene, xylene, and anisole; ester compounds such as methyl acetate, ethyl acetate, propyl acetate, and butyl acetate; ketone compounds such as acetone and methyl ethyl ketone; and dimethylformamide, acetonitrile, dimethyl sulfoxide, alcohols, and water. These polymerization solvents may be used singly, or two or more types of the polymerization solvents may be used in combination.

The reaction temperature in the polymerization reaction by the RAFT method is preferably 40°C or higher and 100°C or lower, more preferably 45°C or higher and 90°C or lower, and still more preferably 50°C or higher and 80°C or lower. A reaction temperature of 40°C or higher is preferable because the polymerization reaction can smoothly proceed, and a reaction temperature of 100°C or lower is preferable because side reactions can be suppressed and restrictions on usable initiators and solvents are relaxed. In addition, the reaction time can be appropriately set according to the monomer and the like to be used, but is preferably 1 hour or longer and 48 hours or shorter, and more preferably 3 hours or longer and 24 hours or shorter. If necessary, the polymerization may be performed in the presence of a chain transfer agent (e.g., alkyl thiol compounds having 2 to 20 carbon atoms). In the production steps, particularly in a situation where a monomer having an acidic group is used, there may be a concern that metal may be mixed due to corrosion of the reactor or the like. In this case, it is preferable to perform production using equipment in which the surface is coated with a fluorine-based resin or the like. Further, in this case, the storage container for the product or the like is preferably a container made of a resin having corrosion resistance or the like. In a case of using a resin container, the container is preferably made of a material in which metal mixing due to dissolution, for example, of fillers or the like is suppressed.

(Vinyl-based monomer)

In the method for producing the polymer, it is preferable to use a vinyl-based monomer (A') having a molecular structure represented by the following structural formula (1').

[Chemical Formula 5]

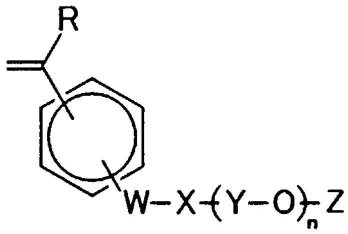

···(1')

In the structural formula (1'), R represents a hydrogen atom or a methyl group, W represents a hydrocarbon group having 6 or less carbon atoms, X represents a heteroatom-containing divalent functional group having 1 or less carbon atoms, Y represents a divalent hydrocarbon group having 6 or less carbon atoms, Z represents a hydrogen atom or an alkyl group having 1 or more and 4 or less carbon atoms, and n represents an integer of 6 or more.
It is noted that R, W, X, Y, Z, and n in the structural formula (1') representing the molecular structure of the vinyl-based monomer (A') are the same as R, W, X, Y, Z, and n in the structural unit of the polymer described above. Therefore, as the description of R, W, X, Y, Z, and n in the vinyl-based monomer (A'), the description regarding R, W, X, Y, Z, and n in the structural unit of the polymer described above can be appropriately referred to.
By using the vinyl-based monomer (A') represented by the structural formula (1'), it is possible to easily obtain the polymer. In addition to having characteristics suitable as an additive for a polishing liquid, the polymer obtained by polymerizing the vinyl-based monomer (A') can be utilized in various applications.
In the structural formula (1'), it is also preferable that W is a methylene group, X is an ether group, and Y is an ethylene group. That is, the vinyl-based monomer preferably has a structure represented by the following structural formula (2).

[Chemical Formula 6]

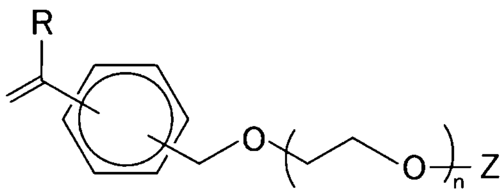

···(2)

(Polishing liquid)

The polymer is suitable, for example, as a component of a polishing liquid. The polishing liquid containing the polymer can be used, for example, for polishing a surface of a silicon wafer. More specifically, the polishing liquid containing the polymer can be used in a polishing step of polishing a surface of a silicon substrate into a high-quality mirror surface, a step of performing chemical mechanical polishing of a silicon wafer, a polishing step of planarizing a surface of an insulating layer and/or a wiring layer in a semiconductor device, and the like in a production process of a semiconductor device.
The polishing liquid may contain abrasive grains in addition to the polymer. As the abrasive grains, it is possible to use at least one or more types of particles selected from the group consisting of known inorganic particles, organic particles, and organic-inorganic composite particles.
Examples of the inorganic particles that can be used include cerium oxide (ceria), fumed silica, fumed alumina, fumed titania, and colloidal silica. In addition, examples of the organic particles that can be used include (meth)acrylic copolymers such as polymethyl methacrylate, polystyrene, polystyrene-based copolymers, polyacetal, polyamides, polycarbonates, polyolefins, polyolefin-based copolymers, and phenoxy resins. As the organic-inorganic composite particles, it is possible to use organic-inorganic composite particles in which a functional group of an organic component and a functional group of an inorganic component are bonded or complexed to such an extent that the particles are not decomposed under conditions used for the polishing liquid composition, such as a composite particle in which the functional group of the organic component and the functional group of the inorganic component are chemically bonded.
The polishing liquid preferably contains one type or two types of abrasive grains selected from the group consisting of ceria and silica. These abrasive grains have a lower hardness than that of alumina or the like, and have an advantage that the occurrence of defects on the polished surface can be suppressed. Thus, the processing accuracy can

be further improved by using these abrasive grains. From the viewpoint of further increasing the removal rate, it is more preferable that the polishing liquid contains abrasive grains made of ceria.

The average particle size of the abrasive grains is not particularly limited, but the average particle size of the abrasive grains may be, for example, within a range of 1 nm or more and 500 nm or less. The average particle size of the abrasive grains is preferably 2 nm or more, and more preferably 3 nm or more from the viewpoint of securing a high removal rate. The upper limit of the average particle size of the abrasive grains is preferably 300 nm or less, and more preferably 100 nm or less from the viewpoint of suppressing occurrence of scratches on the surface of a polishing target. It is noted that the average particle size of the abrasive grains herein means a volume-based median diameter (i.e., d50) of primary particles as measured by a dynamic light scattering method (DLS).

The content of the abrasive grains in the polishing liquid is preferably 0.1 mass% or more, more preferably 1 mass% or more, and still more preferably 2 mass% or more from the viewpoint of achieving a high removal rate. On the other hand, from the viewpoint of improving the smoothness of the polishing target, the content of the abrasive grains in the polishing liquid is preferably 20 mass% or less, more preferably 10 mass% or less, and still more preferably 5 mass% or less.

In configuring the preferred range of the content of the abrasive grains in the polishing liquid, the lower limit and the upper limit of the content of the abrasive grains described above can be arbitrarily combined. For example, the preferred range of the content of the abrasive grains in the polishing liquid may be 0.1 mass% or more and 20 mass%, 1 mass% or more and 10 mass% or less, or 2 mass% or more and 5 mass% or less.

The polishing liquid may contain a solvent. The solvent is preferably an aqueous solvent. Examples of the aqueous solvent include water and a mixed solvent of water and other solvent(s). The other solvent is preferably a solvent compatible with water. Examples of such a solvent include alcohols such as ethanol. The polishing liquid may further contain known additive(s) such as a polishing accelerator, a pH adjusting agent, a surfactant, a chelating agent, or an anticorrosive as long as the effect of the polymer is not impaired.

The concentration of the polymer in the polishing liquid is preferably 0.001 mass% or more, and more preferably 0.05 mass% or more. In addition, the concentration of the polymer in the polishing liquid is preferably 10 mass% or less, and more preferably 5 mass% or less. In configuring the preferred range of the concentration of the polymer in the polishing liquid, the upper limit and the lower limit of the concentration of the polymer described above can be arbitrarily combined. For example, the preferred range of the concentration of the polymer in the polishing liquid may be 0.001 mass% or more and 10 mass% or less, or 0.05 mass% or more and 5 mass% or less.

The polishing liquid is usually prepared as a slurry-like mixture by mixing the respective components by a known method. The viscosity of the polishing liquid at 25°C can be appropriately selected according to a polishing target, a shearing rate during polishing, and the like, and is preferably in a range of 0.1 mPa·s or more and 10 mPa·s or less, and more preferably in a range of 0.5 mPa·s or more and 5 mPa·s or less.

When the polishing liquid is prepared, the polymer may be added to the polishing liquid alone, or may be added to the polishing liquid in a mixture state mixed with other components. Examples of the component to be mixed with the polymer include solvents such as water, an organic solvent, and a mixed solvent of water and an organic solvent. The solvent is preferably capable of dissolving the polymer, and is more preferably water or a mixed solvent of water and an organic solvent soluble in water, and particularly preferably water. Examples of the organic solvent to be used together with water include alcohols such as methanol, ethanol, propanol, and butanol; ketones such as acetone and methyl ethyl ketone; alkylene glycols such as ethylene glycol and propylene glycol; ethers such as ethylene glycol monomethyl ether, propylene glycol monomethyl ether, ethylene glycol dimethyl ether and tetrahydrofuran; esters such as ethylene glycol monomethyl ether acetate and ethyl acetate; amide-based solvents such as N,N-dimethylformamide and N,N-dimethylacetamide; and nitrile-based solvents such as acetonitrile. These organic solvents may be used singly, or two or more types of the organic solvents may be used in combination.

When a mixture of the polymer and a solvent is used for preparation of the polishing liquid, from the viewpoint of bringing the surfaces of the polishing target and the polishing pad into sufficient contact with the polymer, the content of the polymer is preferably 0.001 mass% or more, more preferably 0.01 mass% or more, and still more preferably 0.05 mass% or more with respect to the total mass of the polymer and the solvent in the mixture. In addition, from the viewpoint of suppressing deterioration in handleability due to an excessively high viscosity, the content of the polymer in the mixture is preferably 70 mass% or less, more preferably 60 mass% or less, and still more preferably 50 mass% or less with respect to the total mass of the polymer and the solvent. In configuring the preferred range of the content of the polymer in the mixture, the lower limit and the upper limit of the content of the polymer described above can be arbitrarily combined. For example, the preferred range of the content of the polymer in the mixture may be 0.001 mass% or more and 70 mass% or less, 0.01 mass% or more and 60 mass% or less, or 0.05 mass% or more and 50 mass% or less with respect to the total mass of the polymer and the solvent.

EXAMPLES

Examples of the vinyl-based polymer will be described below. The materials used in the Examples are as follows.

[Vinyl-based monomer (A)]

StPEO-550: vinylbenzyl methoxy poly(oxyethylene)ether (average addition mole number: 13)
StPEO-1000: vinylbenzyl methoxy poly(oxyethylene)ether (average addition mole number: 23)

It is noted that a method for synthesizing StPEO-550 or StPEO-1000 is as follows.

· Method for synthesizing StPEO-550

In a reaction vessel containing 1500 mL of tetrahydrofuran, 143 g of polyoxyethylene monomethyl ether having an average molecular weight of 550 ("UNIOX (registered trademark) M-550" manufactured by NOF CORPORATION) was added. While the reaction vessel was cooled in an ice bath, nitrogen bubbling and stirring of the contents in the reaction vessel were performed. Next, 10 g of sodium hydride was added to the reaction vessel, a tetrahydrofuran solution containing p-chloromethylstyrene (manufactured by AGC SEIMI CHEMICAL CO., LTD.) was further slowly added dropwise, and the mixture was stirred for 24 hours under a nitrogen atmosphere. Thereafter, water and dichloromethane were added to the reaction vessel, and reprecipitation purification was performed to obtain StPEO-550. The purity of the obtained StPEO-550 was checked by liquid chromatography, and the purity of StPEO-550 after purification was 97%.

· Method for synthesizing StPEO-1000

The method for synthesizing StPEO-1000 is the same as the method for synthesizing StPEO-550, except that polyoxyethylene monomethyl ether having an average molecular weight of 1000 ("UNIOX M-1000" manufactured by NOF CORPORATION) was used in place of polyoxyethylene monomethyl ether having an average molecular weight of 550.

[Vinyl-based monomer (B)]

AME-400: methoxypolyethylene glycol monoacrylate (n = 9) ("BLEMMER (registered trademark) AME-400" manufactured by NOF CORPORATION)
TBAM: N-tert-butylacrylamide
NIPAM: N-isopropylacrylamide
BA: n-butyl acrylate

[Radical polymerization initiator]

V-501: 4,4-azobis(4-cyanovaleric acid) (manufactured by FUJIFILM Wako Pure Chemical Corporation)

[Chain transfer agent]

3-MPA: aqueous solution containing 5% 3-mercaptopropionic acid

[RAFT agent]

BM1429: 3-((((1-carboxyethyl)thio)carbonothioyl)thio)propanoic acid ("BM1429" manufactured by BORON MOLECULAR)
Methods for synthesis and analysis of the polymers used in Examples and Comparative Examples are as follows.

(Synthesis of polymer)

[Polymer P1-1]

A test tube with a stirring bar was charged with 9.60 g of pure water, 6.00 g of StPEO-550, 0.08 g of V-501, and 2.55 g of 3-MPE, and the inside of the test tube was sufficiently degassed by nitrogen bubbling. Thereafter, the test tube was heated to 85°C to start polymerization. When 3 hours had elapsed from the start of heating, the test tube was cooled with water to stop the polymerization. Thus, a polymer P1-1 was obtained.

[Polymer P1-2]

The method for synthesizing the polymer P1-2 is the same as the method for producing the polymer P1-1, except that the type of the vinyl-based monomer was changed as shown in Table 1.

[Polymers P1-3 to P1-4]

The method for synthesizing the polymer P1-3 to P1-4 is the same as the method for producing the polymer P1-1 to P1-2, except that a RAFT agent (specifically, BM1429) was used instead of the chain transfer agent.

[Polymer CP1-1]

A 500-mL four-necked round-bottom flask equipped with a stirrer, a thermometer, and a nitrogen inlet tube was charged with 50.00 g of pure water, 75.00 g of AME-400, 0.60 g of V-501, and 4.50 g of 3-MPA, and the inside of the flask was sufficiently degassed by nitrogen bubbling. Thereafter, the flask was heated in a constant-temperature bath set at a temperature of 70°C to start polymerization. When 3 hours had elapsed from the start of heating, the flask was cooled with water to stop the polymerization. Thus, the polymer CP1-1 was obtained.

[Polymer CP1-2]

The method for synthesizing the polymer CP1-2 is the same as the method for producing the polymer CP1-1, except that the amounts of pure water and the radical polymerization initiator added were changed as shown in Table 2 and a RAFT agent (specifically, BM1429) was used instead of the chain transfer agent.

[Polymer P2-1]

A test tube with a stirring bar was charged with 12.00 g of pure water, 6.00 g of StPEO-550 as the first monomer, 0.08 g of V-501, and 0.31 g of BM1429, and the inside of the test tube was sufficiently degassed by nitrogen bubbling. Thereafter, the test tube was heated to 85°C to start first-stage polymerization. When 3 hours had elapsed from the start of heating, the test tube was cooled with water to stop the first-stage polymerization. Next, 0.30 g of BA as the second monomer was added to the test tube, and the inside of the test tube was sufficiently degassed by nitrogen bubbling. Thereafter, the test tube was heated to 90°C to start second-stage polymerization. When 3 hours had elapsed from the start of heating, the test tube was cooled with water to stop the second-stage polymerization. Thus, a polymer P2-1 was obtained.

[Polymers P2-2 to P2-4]

The methods for synthesizing polymers P2-2 to P2-4 are the same as the method for producing the polymer P2-1, except that the type and amount of the second monomer blended were changed as shown in Table 3.

[Polymers P2-5 to P2-8]

The methods for synthesizing polymers P2-5 to P2-8 are the same as the method for producing the polymer P2-1, except that StPEO-1000 was used as the first monomer and the type and amount of the second monomer blended were changed as shown in Table 4.

[Polymers CP2-1 to P2-3]

The methods for synthesizing polymers CP2-1 to CP2-3 are the same as the method for producing the polymer P2-1, except that AME-400 was used instead of StPEO-550 as the first monomer and the type of the second monomer was changed as shown in Table 5.

(Analysis method)

[Measurement of molecular weight]

For the polymers P1-1 to 1-4, CP1-1 to 1-2, P2-1 to P2-8, and CP2-1 to CP2-3, the number average molecular weight Mn and the weight average molecular weight Mw in terms of polymethyl methacrylate were measured under the following conditions by using a gel permeation chromatograph (Model name: "HLC -8320", manufactured by Tosoh Corporation). In addition, the obtained values were used to calculate the dispersity index PDI of the molecular weight, that is, the ratio Mw/Mn of the weight molecular weight average amount Mw to the number average molecular weight Mn. The number average molecular weight Mn, the weight average molecular weight Mw, and the dispersity index PDI of each polymer were as shown in Tables 1 to 5.

· Measurement conditions

Column: TSKgel Super HM-M (manufactured by Tosoh Corporation) × 3 columns
Column temperature: 40°C
Eluent: N,N-dimethylformamide
Standard substance: polymethyl methacrylate
Flow rate: 0.3 mL/min

Next, polishing liquids of Examples and Comparative Examples were prepared using the polymers P1-1 to P1-4, CP1-1 to 1-2, P2-1 to P2-8, and the polymers CP2-1 to CP2-3.

(Examples 1-1 to 1-4, Examples 2-1 to 2-8, Comparative Examples 1-2 to 1-3, and Comparative Examples 2-1 to 2-3)

A colloidal ceria water dispersion ("Nyacol Ce 80/10" manufactured by Nyacol; particle concentration: 10%; average particle diameter: 80 nm) was diluted with water so as to have a predetermined abrasive grain concentration, and one of the polymers P1-1 to P1-4, P2-1 to P2-8, CP1-1 to 1-2, and CP2-1 to CP2-3 obtained by the above-described methods was added so as to have a predetermined polymer concentration. Thus, polishing liquids of Examples 1-1 to 1-4, Examples 2-1 to 2-8, Comparative Examples 1-2 to 1-3, and Comparative Examples 2-1 to 2-3 were obtained.

(Comparative Example 1-1)

A colloidal ceria water dispersion ("Nyacol Ce 80/10" manufactured by Nyacol; particle concentration: 10%; average particle diameter: 80 nm) was diluted with water so as to have a predetermined abrasive grain concentration. Thus, a polishing liquid of Comparative Example 1-1 was obtained.

(Polishing test)

As a polishing target, a blanket wafer having a $SiO_2$ film with a thickness of 1,000 nm formed on a 4-inch silicon substrate was prepared. Next, the polishing target was polished for 4 minutes using each of the polishing liquids of Examples and Comparative Examples under the conditions below. Then, the removal amount (RA) (unit: nm) was calculated by subtracting the thickness of the $SiO_2$ film after polishing from the thickness of the $SiO_2$ film before polishing. The thicknesses of the $SiO_2$ film before and after polishing were measured using a optical interference film thickness meter.

[Polishing conditions]

Polishing tester: EJ-380IN-CH-Y-D (trade name), manufactured by ENGIS JAPAN Corporation
Polishing pad: IC-1000 (trade name), manufactured by NITTA DuPont Incorporated
Platen rotation speed: 60 rpm
Carrier rotation speed: 40 rpm
Supply amount of polishing liquid: 30 g/min
Polishing load: 3 kgf, 7 kgf, or 12 kgf

[Evaluation of removal rate]

To evaluate the removal rate, a ratio RAb/RAa was calculated, as defined by a removal amount RAb when polishing was performed with a polishing load of 12 kgf while using each of the polishing liquids of Examples 1-1 to 1-4, Examples 2-1 to 2-8, Comparative Examples 1-2 to 1-3, and Comparative Examples 2-1 to 2-3, relative to a removal amount RAa when polishing was performed with a polishing load of 12 kgf while using the polishing liquid of Comparative Example 1-1. Then, the removal rate was evaluated based on the value of the ratio RAb/RAa. The value of the ratio RAb/RAa means that the larger the value, the higher the removal rate, which is preferable.
Tables 6 to 10 show the values of RA3, RA7, RA12, the value of the ratio RAb/RAa, and the evaluation results of the removal rate in each of the polishing liquids of Examples and Comparative Examples. The meanings of the symbols shown in the "Removal rate" of Tables 6 to 10 are as follows.
A:

$$RAb/RAa \geq 0.60$$

B:

$$0.60 > RAb/RAa \geq 0.50$$

C:

$$0.50 > RAb/RAa$$

[Evaluation of dishing suppression performance]

To evaluate the dishing suppression performance for each polishing liquid, a ratio RA7/RA3, defined as a removal amount RA7 when polishing was performed with a polishing load of 7 kgf to a removal amount RA3 when polishing was performed with a polishing load of 3 kgf, and a ratio RA12/RA3, defined as a removal amount RA12 when polishing was performed with a polishing load of 12 kgf to the removal amount RA3 when polishing was performed with a polishing load of 3 kgf, were calculated. Then, the dishing suppression performance was evaluated based on the values of the ratio RA7/RA3 and the ratio RA12/RA3.
When the vinyl-based polymer is not added to the polishing liquid, the removal rate usually increases in proportion to the polishing pressure. On the other hand, when the vinyl-based polymer is added to the polishing liquid, the removal rate when polishing is performed at a low polishing pressure may be lower and the removal rate when polishing is performed at a high polishing pressure may be higher, as compared with a polishing liquid free of the vinyl-based polymer. A polishing liquid having such polishing characteristics can provide a favorable polished surface in which dishing of the pattern wafer is suppressed without lowering the removal rate. Therefore, the values of the ratio RA12/RA3 and the ratio RA7/RA3 mean that the larger the values, the more effectively dishing can be suppressed, which is preferable.
Tables 6 to 10 show the values of the ratio RA7/RA3, the ratio RA12/RA3, and the evaluation results of dishing suppression performance in each of the polishing liquids of Examples and Comparative Examples. The meanings of the symbols shown in the "Dishing suppression performance" of Tables 6 to 10 are as follows.

A: RA7/RA3 ≥ 4.0 and RA12/RA3 ≥ 11.5
B: In a case where the above A is not satisfied, and at least one of 4.0 > RA7/RA3 > 3.8 and 11.5 > RA12/RA3 > 10.0 is satisfied.
C: In a case where the above A and B are not satisfied, and at least one of 3.8 ≥ RA7/RA3 > 3.0 and 10.0 ≥ RA12/RA3 > 9.0 is satisfied.
D: In a case where the above A to C are not satisfied, at least one of 3.0 ≥ RA7/RA3 and 9.0 ≥ RA12/RA3 is satisfied.

(Evaluation of storage stability)

Among the polishing liquids of Examples and Comparative Examples, each polishing liquid containing a polymer was dissolved in ion-exchanged water to prepare a sample solution having a concentration of 5%. This sample solution was used to evaluate the storage stability in an acidic environment and in an alkaline environment by the following methods.

[Storage stability in acidic environment]

First, the initial pH and acid value of the sample solution were measured. Next, the sample solution was left to stand at a temperature of 60°C for 1 week. Then, the pH and the acid value of the sample solution after standing were measured, and a change amount ΔpH of the pH after standing with reference to the initial pH and a change amount ΔAV of the acid value after standing with reference to the initial acid value were calculated.

[Storage stability in alkaline environment]

First, the initial pH and acid value of the sample solution were measured. Next, the pH of the sample solution was adjusted to 10.0 using potassium hydroxide. The sample solution after adjusting the pH was left to stand at a temperature of 60°C for 1 week. Hydrochloric acid at the molar number equal to the molar number of potassium hydroxide added at the time of pH adjustment was added to the sample solution after standing, and the pH and the acid value of the sample solution were then measured. Subsequently, a change amount ΔpH of the pH after standing with reference to the initial pH and a change amount ΔAV of the acid value after standing with reference to the initial acid value were calculated.
It is noted that the pH was measured using a pH meter ("D-51" manufactured by HORIBA, Ltd.). In addition, the temperature of the sample solution used for pH measurement was set to 25°C. In the measurement of the acid value, first, an appropriate amount of the sample solution was dissolved in 50 mL of tetrahydrofuran, and a small amount of water was further added to prepare a titration sample. The titration sample was titrated using a 0.1 mol/L KOH-EtOH solution, and the neutralization point was measured to calculate the acid value. Incidentally, an automatic titrator ("COM-A19" manufactured by HIRANUMA Co., Ltd.) was used for the titration.
The storage stability under each environment was evaluated based on the ΔpH and ΔAV under the acidic environment and the ΔpH and ΔAV under the alkaline environment obtained as described above. Tables 6 to 10 show the values of ΔpH and ΔAV, and the evaluation results of the storage stability under each environment for each polishing liquid containing the polymer. It is noted that the meanings of the symbols shown in the "Evaluation" of Tables 6 to 10 are as follows.

A: ΔpH < 0.22 and ΔAV ≤ 1.0
B: In a case where the above A is not satisfied, and at least one of 0.22 ≤ ΔpH ≤ 0.25 and 1.0 < ΔAV ≤ 1.9 is satisfied.
C: In a case where the above A and B are not satisfied, and at least one of 0.25 < ΔpH ≤ 0.35 and 1.9 < ΔAV ≤ 2.5 is satisfied.
D: In a case where the above A to C are not satisfied, and at least one of 0.35 < ΔpH and 2.5 < ΔAV is satisfied.

[Table 1]

(Table 1)

| | | Unit | Polymer P1-1 | Polymer P1-2 | Polymer P1-3 | Polymer P1-4 |
|---|---|---|---|---|---|---|
| Monomer | StPEO-550 | g | 6.00 | - | 6.00 | - |
| | StPEO-1000 | g | - | 6.00 | - | 6.00 |
| | AME400 | g | - | - | - | - |
| Radical polymerization initiator | V-501 | g | 0.08 | 0.08 | 0.08 | 0.08 |
| Solvent | Water | g | 9.60 | 9.60 | 12.00 | 12.00 |
| RAFT agent | BM1429 | g | - | - | 0.31 | 0.31 |
| Chain transfer agent | 3-MPA | g | 2.55 | 2.55 | - | - |
| Number average molecular weight Mn | | - | 6,940 | 7,140 | 5,890 | 5,980 |
| Weight average molecular weight Mw | | - | 15,960 | 15,120 | 6,670 | 6,790 |
| Dispersity index PDI | | - | 2.30 | 2.12 | 1.13 | 1.14 |

[Table 2]

(Table 2)

| | | Unit | Polymer CP1-1 | Polymer CP1-2 |
|---|---|---|---|---|
| Monomer | StPEO-550 | g | - | - |
| | StPEO-1000 | g | - | - |
| | AME-400 | g | 75.00 | 75.00 |
| Radical polymerization initiator | V-501 | g | 0.60 | 0.12 |
| Solvent | Pure water | g | 50.00 | 37.50 |
| RAFT agent | BM1429 | g | - | 1.91 |
| Chain transfer agent | 3-MPA | g | 4.50 | - |
| Number average molecular weight Mn | | - | 2,800 | 7,000 |
| Weight average molecular weight Mw | | - | 3,480 | 7,740 |
| Dispersity index PDI | | - | 1.24 | 1.11 |

[Table 3]

(Table 3)

| | | Unit | Polymer P2-1 | Polymer P2-2 | Polymer P2-3 | Polymer P2-4 |
|---|---|---|---|---|---|---|
| First monomer | StPEO-550 | g | 6.00 | 6.00 | 6.00 | 6.00 |
| | StPEO-1000 | g | - | - | - | - |
| | AME-400 | g | - | - | - | - |
| Radical polymerization initiator | V-501 | g | 0.08 | 0.08 | 0.08 | 0.08 |
| Solvent | Pure water | g | 12.00 | 12.00 | 12.00 | 12.00 |
| RAFT agent | BM1429 | g | 0.31 | 0.31 | 0.31 | 0.31 |
| Second monomer | NIPAM | g | - | - | 0.30 | 0.60 |
| | TBAM | g | - | 0.30 | - | - |
| | BA | g | 0.30 | - | - | - |
| Number average molecular weight Mn | | - | 6,710 | 7,400 | 7,160 | 7,720 |
| Weight average molecular weight Mw | | - | 7,920 | 9,700 | 8,130 | 8,930 |
| Dispersity index PDI | | - | 1.18 | 1.31 | 1.14 | 1.16 |

[Table 4]

(Table 4)

| | | Unit | Polymer P2-5 | Polymer P2-6 | Polymer P2-7 | Polymer P2-8 |
|---|---|---|---|---|---|---|
| First monomer | StPEO-550 | g | - | - | - | - |
| | StPEO-1000 | g | 6.00 | 6.00 | 6.00 | 6.00 |
| | AME-400 | g | - | - | - | - |
| Radical polymerization initiator | V-501 | g | 0.08 | 0.08 | 0.08 | 0.08 |
| Solvent | Pure water | g | 12.00 | 12.00 | 12.00 | 12.00 |
| RAFT agent | BM1429 | g | 0.31 | 0.31 | 0.31 | 0.31 |
| Second monomer | NIPAM | g | - | - | 0.30 | 0.60 |
| | TBAM | g | - | 0.30 | - | - |
| | BA | g | 0.30 | - | - | - |
| Number average molecular weight Mn | | - | 7,890 | 9,180 | 8,430 | 8,550 |
| Weight average molecular weight Mw | | - | 10,180 | 11,660 | 10,120 | 10,600 |
| Dispersity index PDI | | - | 1.29 | 1.27 | 1.20 | 1.24 |

[Table 5]

(Table 5)

| | | Unit | Polymer CP2-1 | Polymer CP2-2 | Polymer CP2-3 |
|---|---|---|---|---|---|
| First monomer | StPEO-550 | g | - | - | - |
| | StPEO-1000 | g | - | - | - |
| | AME-400 | g | 10.00 | 10.00 | 10.00 |
| Radical polymerization initiator | V-501 | g | 0.02 | 0.02 | 0.02 |
| Solvent | Pure water | g | 5.00 | 5.00 | 5.00 |
| RAFT agent | BM1429 | g | 0.25 | 0.25 | 0.25 |
| Second monomer | NIPAM | g | - | - | 0.50 |
| | TBAM | g | - | 0.50 | - |
| | BA | g | 0.50 | - | - |
| Number average molecular weight Mn | | - | 7,790 | 7,710 | 7,700 |
| Weight average molecular weight Mw | | - | 8,340 | 8,250 | 8,240 |
| Dispersity index PDI | | - | 1.07 | 1.07 | 1.07 |

[Table 6]

(Table 6)

| | Unit | Example 1-1 | Example 1-2 | Example 1-3 | Example 1-4 |
|---|---|---|---|---|---|
| Vinyl-based polymer | - | Polymer P1-1 | Polymer P1-2 | Polymer P1-3 | Polymer P1-4 |
| Abrasive grains | - | Ceria | Ceria | Ceria | Ceria |

(continued)

| | | | Unit | Example 1-1 | Example 1-2 | Example 1-3 | Example 1-4 |
|---|---|---|---|---|---|---|---|
| Polishing test | RA3 | | nm | 18.6 | 15.3 | 15.3 | 12.9 |
| | RA7 | | nm | 64.8 | 56.0 | 65.4 | 66.0 |
| | RA12 | | nm | 175.8 | 146.3 | 159.2 | 153.9 |
| | RAb/RAa | | - | 0.56 | 0.47 | 0.51 | 0.49 |
| | Removal rate | | - | B | C | B | C |
| | RA7/RA3 | | - | 3.48 | 3.66 | 4.26 | 5.12 |
| | RA12/RA3 | | - | 9.44 | 9.57 | 10.38 | 11.95 |
| | Dishing suppression performance | | - | C | C | B | A |
| Storage stability | Acidic | ΔpH | - | 0.04 | < 0.01 | 0.24 | 0.35 |
| | | ΔAV | - | 0.28 | < 0.1 | 1.89 | 1.64 |
| | | Evaluation | - | A | A | B | B |
| | Alkaline | ΔpH | - | 0.09 | 0.16 | 0.30 | 0.35 |
| | | ΔAV | - | 1.07 | 0.88 | 1.76 | 1.63 |
| | | Evaluation | - | B | A | B | B |

[Table 7]

(Table 7)

| | | | Unit | Comparative Example 1-1 | Comparative Example 1-2 | Comparative Example 1-3 |
|---|---|---|---|---|---|---|
| Vinyl-based polymer | | | - | None | Polymer CP1-1 | Polymer CP 1-2 |
| Abrasive grains | | | - | Ceria | Ceria | Ceria |
| Polishing test | RA3 | | nm | 27.4 | 20.0 | 21.1 |
| | RA7 | | nm | 115.8 | 64.8 | 107.7 |
| | RA12 | | nm | 314.0 | 204.1 | 207.2 |
| | RAb/RAa | | - | 1 | 0.65 | 0.66 |
| | Removal rate | | - | - | A | A |
| | RA7/RA3 | | - | 4.23 | 3.24 | 5.10 |
| | RA12/RA3 | | - | 11.46 | 10.2 | 9.82 |
| | Dishing suppression performance | | - | - | B | C |
| Storage stability | Acidic | ΔpH | - | - | 0.37 | 0. 17 |
| | | ΔAV | - | - | 7.29 | 4.60 |
| | | Evaluation | - | - | D | D |
| | Alkaline | ΔpH | - | - | 0.30 | 0.33 |
| | | ΔAV | - | - | 5.89 | 3.44 |
| | | Evaluation | - | - | C | C |

[Table 8]

(Table 8)

| | | | Unit | Example 2-1 | Example 2-2 | Example 2-3 | Example 2-4 |
|---|---|---|---|---|---|---|---|
| Vinyl-based polymer | | | - | Polymer P2-1 | Polymer P2-2 | Polymer P2-3 | Polymer P2-4 |
| Abrasive grains | | | - | Ceria | Ceria | Ceria | Ceria |
| Polishing test | RA3 | | nm | 11.5 | 13.7 | 16.4 | 13.2 |
| | RA7 | | nm | 55.6 | 63.7 | 68.3 | 68.3 |
| | RA12 | | nm | 150.7 | 141.3 | 204.1 | 147.6 |
| | RAb/RAa | | - | 0.48 | 0.45 | 0.65 | 0.47 |
| | Removal rate | | - | C | C | A | C |
| | RA7/RA3 | | - | 4.83 | 4.65 | 4.15 | 5.19 |
| | RA12/RA3 | | - | 13.1 | 10.31 | 12.42 | 11.22 |
| | Dishing suppression performance | | - | A | B | A | B |
| Storage stability | Acidic | ΔpH | - | 0.10 | 0.04 | 0.10 | 0.09 |
| | | ΔAV | - | 1.51 | 0.85 | 0.86 | 0.53 |
| | | Evaluation | - | B | A | A | A |
| | Alkaline | ΔpH | - | 0.14 | 0.15 | 0.09 | 0.06 |
| | | ΔAV | - | 1.03 | 1.12 | 0.89 | 0.63 |
| | | Evaluation | - | B | B | A | A |

[Table 9]

(Table 9)

| | | Unit | Example 2-5 | Example 2-6 | Example 2-7 | Example 2-8 |
|---|---|---|---|---|---|---|
| Vinyl-based polymer | | - | Polymer P2-5 | Polymer P2-6 | Polymer P2-7 | Polymer P2-8 |
| Abrasive grains | | - | Ceria | Ceria | Ceria | Ceria |
| Polishing test | RA3 | nm | 17.5 | 13.2 | 11.2 | 13.7 |
| | RA7 | nm | 66.0 | 62.5 | 57.9 | 78.7 |
| | RA12 | nm | 144.4 | 138.2 | 144.4 | 135 |
| | RAb/RAa | - | 0.46 | 0.44 | 0.46 | 0.43 |
| | Removal rate | - | C | C | C | C |
| | RA7/RA3 | - | 3.76 | 4.75 | 5.15 | 5.75 |
| | RA12/RA3 | - | 8.24 | 10.51 | 12.86 | 9.86 |
| | Dishing suppression performance | - | C | B | A | C |

(continued)

| | | | Unit | Example 2-5 | Example 2-6 | Example 2-7 | Example 2-8 |
|---|---|---|---|---|---|---|---|
| Storage stability | Acidic | ΔpH | - | 0.14 | 0.20 | 0.06 | 0.01 |
| | | ΔAV | - | 0.94 | 0.96 | 0.86 | 0.31 |
| | | Evaluation | - | A | A | A | A |
| | Alkaline | ΔpH | - | 0.10 | 0.14 | 0.16 | 0.11 |
| | | ΔAV | - | 1.36 | 1.06 | 0.81 | 0.38 |
| | | Evaluation | - | B | B | A | A |

[Table 10]

(Table 10)

| | | | Unit | Comparative Example 2-1 | Comparative Example 2-2 | Comparative Example 2-3 |
|---|---|---|---|---|---|---|
| Vinyl-based polymer | | | - | Polymer CP2-1 | Polymer CP2-2 | Polymer CP2-3 |
| Abrasive grains | | | - | Ceria | Ceria | Ceria |
| Polishing test | RA3 | | nm | 17.8 | 17 | 14.9 |
| | RA7 | | nm | 71.8 | 94.9 | 69.8 |
| | RA12 | | nm | 207.2 | 188.4 | 189.7 |
| | RAb/RAa | | - | 0.54 | 0.60 | 0.60 |
| | Removal rate | | - | B | A | A |
| | RA7/RA3 | | - | 4.03 | 5.59 | 4.68 |
| | RA12/RA3 | | - | 9.52 | 11.09 | 12.72 |
| | Dishing suppression performance | | - | C | B | A |
| Storage stability | Acidic | ΔpH | - | 0.56 | 0.04 | 0.12 |
| | | ΔAV | - | 4.72 | 1.69 | 2.11 |
| | | Evaluation | - | D | B | C |
| | Alkaline | ΔpH | - | 0.22 | 0.28 | 0.19 |
| | | ΔAV | - | 2.49 | 2.21 | 1.93 |
| | | Evaluation | - | B | C | C |

As shown in Table 1, the vinyl-based polymers P1-1 to P1-4 contained in the polishing liquids of Examples 1-1 to 1-4, respectively, are composed of structural unit (A) derived from the vinyl-based monomer (A) (more specifically, StPEO-550 or StPEO-1000) represented by the structural formula (1). Therefore, as shown in Table 6, these polishing liquids exhibited small changes in the pH and acid value and exhibited excellent storage stability in both the cases of storage in an acidic environment and storage in an alkaline environment.

Similarly, as shown in Tables 3 and 4, the vinyl-based polymers P2-1 to P2-8 contained in the polishing liquids of Examples 2-1 to 2-8, respectively, are composed of structural unit derived from the vinyl-based monomer (A) (more specifically, StPEO-550 or StPEO-1000) represented by the structural formula (1) and structural unit derived from the vinyl-based monomer (B) different from the vinyl-based polymer (A). Therefore, as shown in Tables 8 and 9, these polishing liquids exhibited small changes in the pH and acid value and exhibited excellent storage stability in both the cases of storage in an acidic environment and storage in an alkaline environment.

In contrast, as shown in Table 2, the vinyl-based polymers CP1-1 to CP1-2 contained in the polishing liquids of Comparative Examples 1-2 to 1-3, respectively, are composed of structural unit derived from vinyl-based monomer other

than the vinyl-based monomer (A). Therefore, as shown in Table 7, these polishing liquids were inferior in storage stability as compared with the polishing liquids of Examples.

Similarly, as shown in Table 5, the vinyl-based polymers CP2-1 to CP2-2 contained in the polishing liquids of Comparative Examples 2-1 to 2-3, respectively, do not contain a structural unit (A) derived from a vinyl-based monomer other than the vinyl-based monomer (A). Therefore, as shown in Table 10, these polishing liquids were inferior in storage stability as compared with the polishing liquids of Examples.

Aspects of the vinyl-based polymer, the method for producing the vinyl-based polymer, the polishing liquid, and the vinyl-based monomer have been described above based on the Examples. However, the specific aspects of the vinyl-based polymer, the method for producing the vinyl-based polymer, the polishing liquid, and the vinyl-based monomer according to the present invention are not limited to those of the Examples, and the configuration may be appropriately modified without departing from the spirit of the present invention.

## Claims

1. A vinyl-based polymer comprising a structural unit (A) derived from a vinyl-based monomer (A) represented by the following structural formula (1):

[Chemical Formula 1]

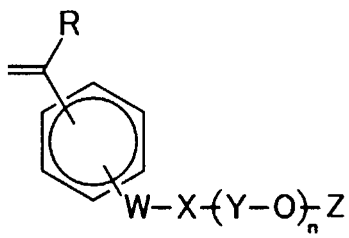

···(1)

wherein in the structural formula (1), R represents a hydrogen atom or a methyl group, W represents a divalent hydrocarbon group having 6 or less carbon atoms, X represents a heteroatom-containing divalent functional group having 1 or less carbon atoms, Y represents a divalent hydrocarbon group having 6 or less carbon atoms, Z represents a hydrogen atom or an alkyl group having 1 or more and 4 or less carbon atoms, and n represents an integer of 4 or more.

2. The vinyl-based polymer according to claim 1, wherein the vinyl-based polymer is composed of the structural unit (A), and the structural unit (A) is derived from a vinyl-based monomer (A') represented by the following structural formula (1'):

[Chemical Formula 2]

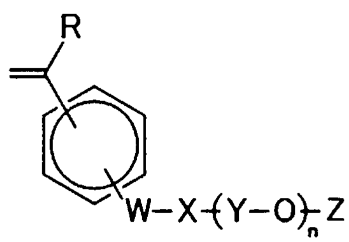

···(1')

wherein in the structural formula (1'), R represents a hydrogen atom or a methyl group, W represents a divalent hydrocarbon group having 6 or less carbon atoms, X represents a heteroatom-containing divalent functional group having 1 or less carbon atoms, Y represents a divalent hydrocarbon group having 6 or less carbon atoms, Z represents a hydrogen atom or an alkyl group having 1 or more and 4 or less carbon atoms, and n represents an integer of 6 or more.

3. The vinyl-based polymer according to claim 1, wherein the vinyl-based polymer comprises the structural unit (A) and a

structural unit (B) derived from a vinyl-based monomer (B) different from the vinyl-based monomer (A).

**4.** The vinyl-based polymer according to claim 3, wherein the vinyl-based polymer comprises the structural unit (B) having an SP value of 17 $(J/cm^3)^{0.5}$ or more and 25 $(J/cm^3)^{0.5}$ or less as calculated by Fedors' estimation method.

**5.** The vinyl-based polymer according to claim 3, wherein the vinyl-based monomer (B) comprises a (meth)acrylamide-based monomer.

**6.** The vinyl-based polymer according to claim 3, wherein the vinyl-based polymer is a block copolymer having a polymer block (A) containing the structural unit (A) and a polymer block (B) containing the structural unit (B).

**7.** The vinyl-based polymer according to claim 6, wherein the polymer block (B) is composed of the structural unit (B) having an SP value of 17 $(J/cm^3)^{0.5}$ or more and 25 $(J/cm^3)^{0.5}$ or less as calculated by Fedors' estimation method.

**8.** The vinyl-based polymer according to claim 6, wherein the vinyl-based polymer is a diblock copolymer having the polymer block (A) and the polymer block (B), and a ratio of the polymer block (A) to a total of the polymer block (A) and the polymer block (B) is 0.1 mass% or more and 99.9 mass% or less.

**9.** The vinyl-based polymer according to claim 1, wherein the vinyl-based polymer has a number average molecular weight Mn of 1,000 or more and 1,000,000 or less.

**10.** The vinyl-based polymer according to claim 1, wherein a dispersity index PDI, as expressed by the following equation (I), of the vinyl-based polymer is 2.0 or less:

$$\text{PDI} = \text{weight average molecular weight Mw/number average molecular weight Mn} \cdots \text{(I)}.$$

**11.** A method for producing the vinyl-based polymer according to any one of claims 1 to 10, the method comprising polymerizing one or more types of monomers including the vinyl-based monomer (A) by a living radical polymerization method to produce the vinyl-based polymer.

**12.** The method according to claim 11, wherein the living radical polymerization method is a reversible addition-fragmentation chain transfer polymerization method.

**13.** A polishing liquid comprising the vinyl-based polymer according to any one of claims 1 to 10.

**14.** The polishing liquid according to claim 13, wherein the polishing liquid is configured to be used for chemical mechanical polishing.

**15.** The polishing liquid according to claim 14, wherein the polishing liquid is configured to be used for planarization of a surface of an insulating layer and/or a wiring layer in a semiconductor device.

**16.** The polishing liquid according to claim 15, wherein the polishing liquid comprises one or two types of abrasive grains selected from the group consisting of ceria and silica.

**17.** A vinyl-based monomer represented by the following structural formula (1'):

[Chemical Formula 3]

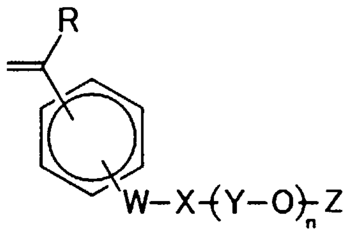

···(1')

wherein in the structural formula (1'), R represents a hydrogen atom or a methyl group, W represents a hydrocarbon group having 6 or less carbon atoms, X represents a heteroatom-containing divalent functional group having 1 or less carbon atoms, Y represents a divalent hydrocarbon group having 6 or less carbon atoms, Z represents an alkyl group having 1 or more and 4 or less carbon atoms, and n represents an integer of 6 or more.

## INTERNATIONAL SEARCH REPORT

International application No.
**PCT/JP2024/017333**

### A. CLASSIFICATION OF SUBJECT MATTER

***C08F 290/06***(2006.01)i; ***B24B 37/00***(2012.01)i; ***C08F 112/14***(2006.01)i; ***C08F 212/14***(2006.01)i; ***C08F 222/38***(2006.01)i; ***C08F 297/00***(2006.01)i; ***C08F 299/02***(2006.01)i; ***C09K 3/14***(2006.01)i; ***H01L 21/304***(2006.01)i
FI: C08F290/06; H01L21/304 622C; C08F112/14; C09K3/14 550Z; B24B37/00 H; C08F212/14; C08F222/38; C08F297/00; C08F299/02

According to International Patent Classification (IPC) or to both national classification and IPC

### B. FIELDS SEARCHED

Minimum documentation searched (classification system followed by classification symbols)

C08F290/06; B24B37/00; C08F112/14; C08F212/14; C08F222/38; C08F297/00; C08F299/02; C09K3/14; H01L21/304

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

CAplus/REGISTRY (STN)

### C. DOCUMENTS CONSIDERED TO BE RELEVANT

| Category* | Citation of document, with indication, where appropriate, of the relevant passages | Relevant to claim No. |
|---|---|---|
| X<br>A | JP 53-79983 A (KURARAY CO., LTD.) 14 July 1978 (1978-07-14)<br>claims, examples 1, 2 | 1-3, 9-12, 17<br>4-8, 13-16 |
| X<br>A | JP 11-140133 A (ASAHI DENKA KOGYO KK) 25 May 1999 (1999-05-25)<br>claims 1, 2, production examples 1-3, examples | 1-3, 6, 8-12, 17<br>4-5, 7, 13-16 |
| X<br>A | JP 2005-213438 A (TAISHO PHARMACEUTICAL CO., LTD.) 11 August 2005 (2005-08-11)<br>claim 1, examples 1, 2 | 1-3, 6, 8-12, 17<br>4-5, 7, 13-16 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **17 July 2024** | **30 July 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**

International application No. **PCT/JP2024/017333**

**C. DOCUMENTS CONSIDERED TO BE RELEVANT**

| Category* | Citation of document, with indication, where appropriate, of the relevant passages | Relevant to claim No. |
|---|---|---|
| X<br>A | JP 2001-64123 A (KAO CORPORATION) 13 March 2001 (2001-03-13)<br>claims 1, 2, paragraphs [0007]-[0011], examples | 1-2, 9-12, 17<br>3-8, 13-16 |
| X<br>A | JP 2013-119557 A (SUMITOMO RUBBER INDUSTRIES, LTD.) 17 June 2013 (2013-06-17)<br>claim 1, examples | 1-3, 9-12, 17<br>4-8, 13-16 |
| X<br>A | JP 2013-104057 A (SUMITOMO RUBBER INDUSTRIES, LTD.) 30 May 2013 (2013-05-30)<br>claim 1, examples | 1-3, 9-12, 17<br>4-8, 13-16 |
| X<br>A | JP 2013-119558 A (SUMITOMO RUBBER INDUSTRIES, LTD.) 17 June 2013 (2013-06-17)<br>claim 1, examples | 1-3, 9-12, 17<br>4-8, 13-16 |
| X<br>A | JP 2012-224768 A (SUMITOMO RUBBER INDUSTRIES, LTD.) 15 November 2012 (2012-11-15)<br>claim 1, examples | 1-3, 9-12, 17<br>4-8, 13-16 |
| X<br>A | JP 2012-172138 A (SUMITOMO RUBBER INDUSTRIES, LTD.) 10 September 2012 (2012-09-10)<br>claim 1, examples | 1-3, 9-12, 17<br>4-8, 13-16 |
| X<br>A | JP 2012-167206 A (SUMITOMO RUBBER INDUSTRIES, LTD.) 06 September 2012 (2012-09-06)<br>claim 1, examples | 1-3, 9-12, 17<br>4-8, 13-16 |
| X<br>A | JP 2007-236317 A (JAPAN SCIENCE AND TECHNOLOGY AGENCY) 20 September 2007 (2007-09-20)<br>claim 1, production example 3 | 1-3, 9-12, 17<br>4-8, 13-16 |
| X<br>A | WO 2011/111460 A1 (JAPAN SCIENCE AND TECHNOLOGY AGENCY) 15 September 2011 (2011-09-15)<br>claim 1, production example 2 | 1-3, 9-12, 17<br>4-8, 13-16 |
| X<br>A | JP 2011-184377 A (JAPAN SCIENCE AND TECHNOLOGY AGENCY) 22 September 2011 (2011-09-22)<br>claim 1, production example 3 | 1-3, 9-12, 17<br>4-8, 13-16 |

Form PCT/ISA/210 (second sheet) (July 2022)

**INTERNATIONAL SEARCH REPORT**
**Information on patent family members**

International application No.
**PCT/JP2024/017333**

| Patent document cited in search report | Publication date (day/month/year) | Patent family member(s) | Publication date (day/month/year) |
|---|---|---|---|
| JP 53-79983 A | 14 July 1978 | (Family: none) | |
| JP 11-140133 A | 25 May 1999 | (Family: none) | |
| JP 2005-213438 A | 11 August 2005 | (Family: none) | |
| JP 2001-64123 A | 13 March 2001 | (Family: none) | |
| JP 2013-119557 A | 17 June 2013 | (Family: none) | |
| JP 2013-104057 A | 30 May 2013 | (Family: none) | |
| JP 2013-119558 A | 17 June 2013 | (Family: none) | |
| JP 2012-224768 A | 15 November 2012 | (Family: none) | |
| JP 2012-172138 A | 10 September 2012 | (Family: none) | |
| JP 2012-167206 A | 06 September 2012 | (Family: none) | |
| JP 2007-236317 A | 20 September 2007 | (Family: none) | |
| WO 2011/111460 A1 | 15 September 2011 | (Family: none) | |
| JP 2011-184377 A | 22 September 2011 | (Family: none) | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2017017177 A


### Non-patent literature cited in the description


- *Polymer Engineering & Science*, 1974, vol. 14 (2), 147-154